# EUROPEAN PATENT SPECIFICATION

(11) **EP 0 325 656 B1**
(45) Date of publication and mention of the grant of the patent: **17.02.1993**
(21) Application number: 87902143.4
(22) Date of filing: 24.03.1987
(51) Int. Cl.: A61B 6/03

(54) **RADIATION TOMOGRAPH**
STRAHLUNGSTOMOGRAPH
TOMOGRAPHE A RADIATIONS

(30) Priority: 24.03.1986 JP 65332/86
(43) Date of publication of application: 02.08.1989
(73) Proprietor: YOKOGAWA MEDICAL SYSTEMS, LTD, Tokyo 190 (JP)
(72) Inventor: MATSUMURA, Shigeru, 6-chome Tachikawa-shi Tokyo 190 (JP)
(74) Representative: Henkel, Feiler, Hänzel & Partner
(86) International application number: JP8700176
(87) International publication number: WO8705791

(56) References cited:
- EP-A- 0 008 337
- DE-A- 2 722 964
- JP-A- 5 234 690
- JP-A- 5 458 393
- US-A- 4 015 129
- US-A- 4 052 619
- US-A- 4 157 472

## Description

This invention relates to an X-ray computerized tomographic device that collects radiation projection data from multiple directions along the sections of a subject, and reconfigures tomographic images based on such collected data. More specifically, this invention provides an improved means to collect radiation projection data.

It has been commonly known that an X-ray computerized tomographic device (or simply a CT device) reconfigures the tomographic image of a subject by radiating an X-ray beam onto a subject from multiple angles along the sections of the subject to collect projection data (or scan data) for subsequent scan data processing on a computer.

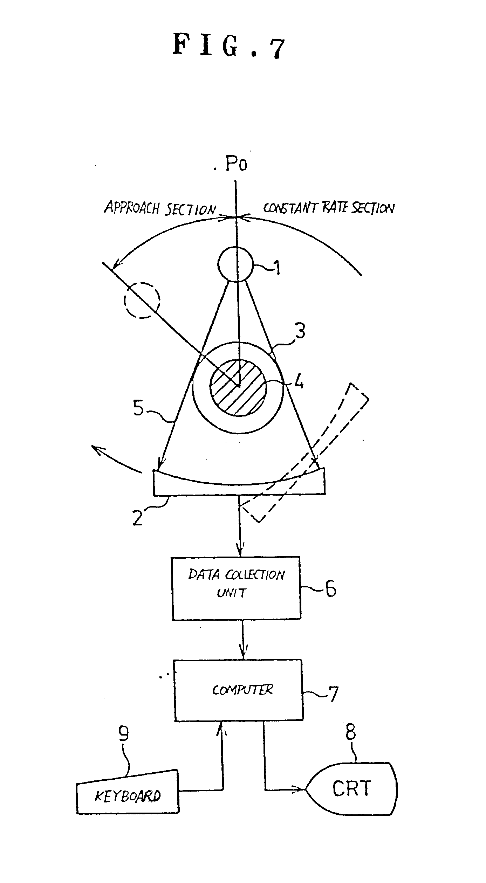

Figure 7 is a block diagram showing the major parts of such a CT device. In Figure 7, an X-ray tube 1 is installed opposite to a detector 2 with a tomographic area 3 located in between. These parts are installed on a gantry (not illustrated) that maintains the above positional relationships. The X-ray tube 1 and the detector 2 rotate around a subject 4 placed in the tomographic area 3 under the control of a gantry controller (not shown in the diagram). The detector 2 is equipped with several hundred detection device channels arranged in a circular arc to detect X-ray 5 in a fan beam form radiated from the x-ray tube 1 passing through the tomographic area 3. A data collection unit 6 is provided with the means to integrate signals detected by the detector 2 for a specific duration of time, and collects these signals at a specific timing, amplifies the collected signals, and converts the output signals into digital data for transfer to the next stage. A computer 7 is equipped with means for storing the data transferred from the data collection unit 6, and operates the image reconfiguration by using the stored data, displays images on a cathode ray tube (CRT) 8 according to the operation results, and sends specific signals to individual sections of the CT device according to signals received from a keyboard 9.

In the above configuration, the CT device rotates the X-ray tube 1 and the detector 2 in a specific direction (clockwise for example), radiates X-ray beams to the subject 4 at multiple predetermined angular positions (view positions or sampling points), and then collects the scan data. When collecting the scan data, this device completes gantry acceleration and sets a constant speed rotation before reaching a reference angular position P₀ after moving over an approach section. The CT device starts collecting scan data when X-ray tube 1 passes over the reference angular position P₀ and continues scanning until it reaches the overlapped section when the first rotation of scan data collection is completed. The angle of gantry rotation in this scan is an aggregate of the angle corresponding to the approach section, the angle corresponding to the deceleration section travel until the rotation stops, and the angle corresponding to overlapped section plus 360^{o}. For the next scan, the section (slice surface) of the subject is changed and the gantry is rotated counterclockwise at the same angles as the previous scan so that scan data can be collected from each sampling point. Then, the above operation is repeated to collect scan data on the desired slice surfaces.

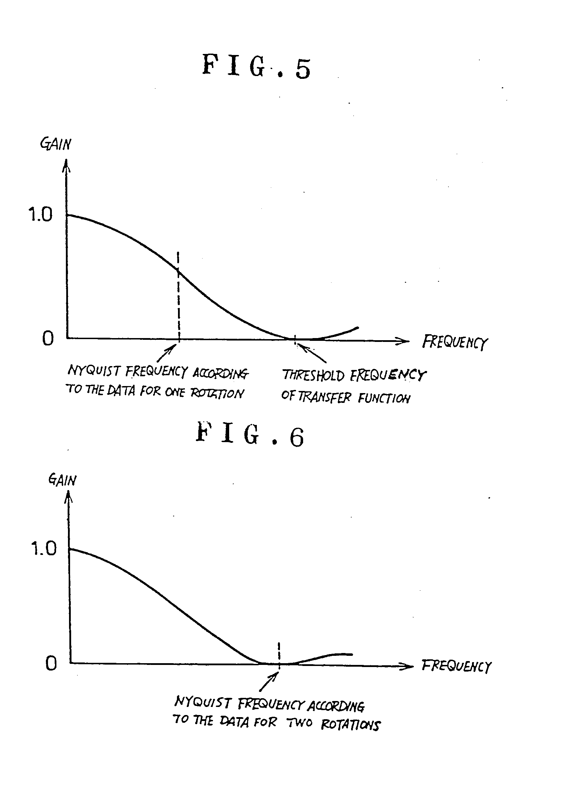

The general method of collecting scan data is described above. To improve the quality of reconfigured images, the following scan methods may be used:
One method uses clockwise and counterclockwise scans to collect scan data for two rotations; the images reconfigured according to respective scan data are added and averaged for display. This enables an S/N ratio equivalent to twice the X-ray dosage to be obtained. The other method is used to collect scan data by interlacing views between clockwise and counterclockwise rotations to reconfigure images based on the collected data. In this case, the view-interlacing collects, data at sampling points X₁, X₃, ..., X₁₅ during a clockwise rotation, and at points X₁₆, X₁₄, ..., X₂ during a counterclockwise rotation as shown in Figure 6. When using such view-interlacing, the creation of artifacts due to insufficient views can be reduced while improving the image quality. However, these scan methods use scan intervals between the clockwise and counterclockwise rotations that accompanies the directional switching of gantry rotation. This may reduce the resolution of reconfigured images, and more artifacts may be created due to the movement of the subject body. Moreover, for scanning using view-interlacing, gantry rotation or sampling points control may become complicated.

It is an object of this invention to overcome the disadvantages of the prior art by providing an X-ray computerized tomographic device that can provide images of good quality without complicating gantry rotation and scan data sampling control. Another object of this invention is to provide an X-ray computerized tomographic device that enable multiple methods of scanning without complicating a gantry rotation and scan data sampling control.

To solve this object the present invention provides an X-rays computerized tomographic device as specified in claim 1.

Summarizing up, the X-ray computerized tomographic device based on this invention is characterized by:
- Setting sampling points of N·M - 1 (N and M being positive integers) around a subject;
- Scanning the sampling points around by skipping M - 1 point and continuously repeating such scanning for M times; and
- Collecting the scan data at every sampling point.

The invention will be explained in more detail with reference to the drawings, in which:
Figure 1 shows a block diagram of a CT device of a typical application mode of this invention,
Figure 2 shows the relationship between a rotary position signal of the gantry and scan data sampling,
Figure 3 shows a schematic diagram of the sampling point arrangement in a preferred application mode of this invention,
Figure 4 explains an overlapped scan,
Figure 5 and 6 show the transfer function of dislocation in the direction of rotation in a continuous X-ray CT device,
Figure 7 shows a block diagram of an approximate configuration of a CT device based on prior art, and

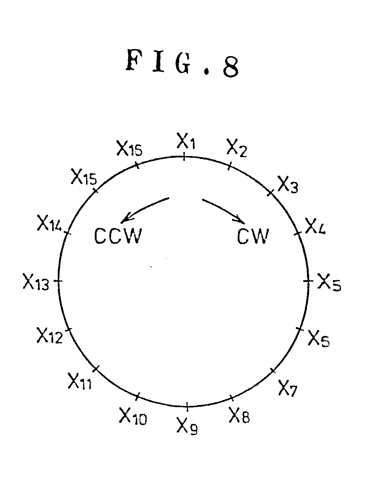

Figure 8 is a schematic diagram showing the scan data sampling of a CT device based on prior art.

The preferred application modes of this invention are described in detail by using examples and referring to the accompanying drawings.

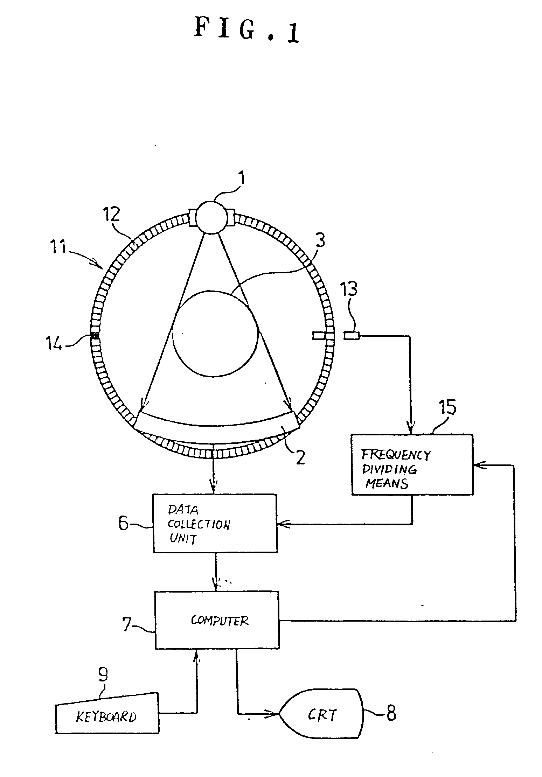

Figure 1 shows a conceptual block diagram of the major parts of a CT device that reflects a preferred application mode of this invention.

In Figure 1, an X-ray tube 1 which irradiates a continuous X-ray beam is installed opposite a detector 2 on the gantry frame (not shown in figure 1) with a tomographic area 3 located in between. On the gantry, a marker device 12 of N·M - 1 units of a rotary encoder 11 is installed. The gantry is controlled by a gantry controller (not shown in figure 1) to rotate continuously around the tomographic area 3 in a specific direction for a minimum of M times. The rotary position of the gantry at this time is detected by a fixed marker detector 13 of the rotary encoder 11. The rotary encoder 11 may have a configuration in which a marker device group 12 is fixed, while the marker detector 13 rotates with the gantry. Among the marker device group 12, an absolute position marker device 14 is installed to detect the rotation reference position of the X-ray tube 1 and the detector 2. An output signal from the marker detector 13 is sent to a frequency divider circuit 15, which divides it at a rate of 1/M and sends it to a data collection unit 6 as a scan data sampling instruction signal. The data collection unit 6 collects scan data from the detector 2 in synchronization with the sampling instruction signal and transfers the signal to a computer 7 after the specified processing is executed. The computer 7 reconfigures an image based on the data and provides the frequency divider circuit 15 with the dividing ratio set from a keyboard 9.

The scan data collection operation of a CT device having the above configuration is described as follows:
For the sake of descriptive convenience, let N=8, M=2.

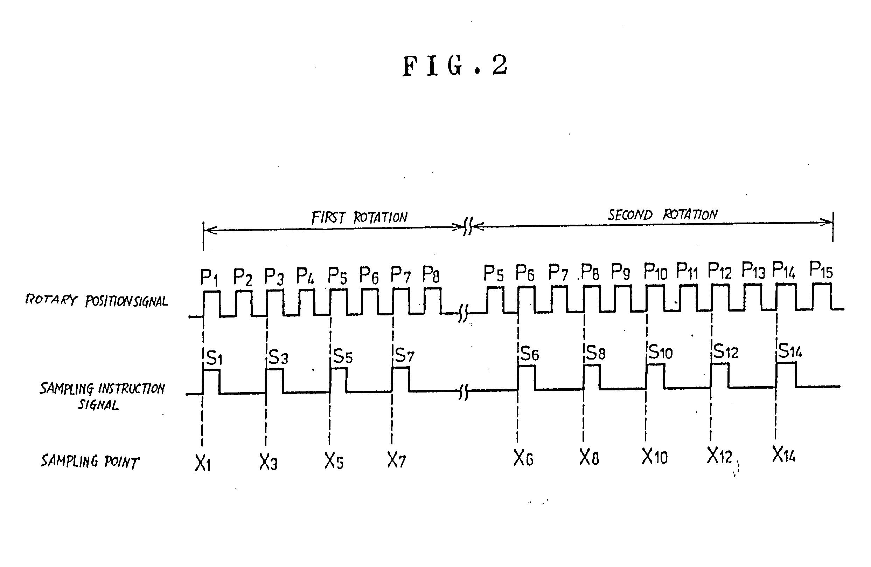

Therefore, the number of the rotary encoder marker device is 15 and the dividing ratio of the frequency divider circuit 15 is 1/2. In an actual device, a value of several hundreds is set for N and M is an integer significantly smaller than N. In this following description, the marker devices are called M₁, M₂, ..., M₁₅. Moreover, absolute position marker device 14 is called marker device M'₁. The gantry is controlled by the gantry controller as it rotates clockwise, for example, at least two times. During these two rotations, the marker detector 13 outputs rotary position signals P₁, P₂, ..., P₁₅ twice via marker devices M₁, M₂, ..., M₁₅ as shown in Figure 2. The frequency divider circuit 15 outputs the sampling instruction signal after dividing the rotary position signals into 1/2.

Accordingly, at the end of the first gantry rotation, sampling instruction signals S₁, S₃, S₅, S₇, ..., S₁₅ are output to match rotary position signals P₁. P₃, P₅, P₇, ..., P₁₅. At the end of the second rotation, sampling instruction signals S₂, S₄, S₆, S₈, ..., S₁₄ are output to match rotary position signals P₂, P₄, P₆, P₈, ..., P₁₄.

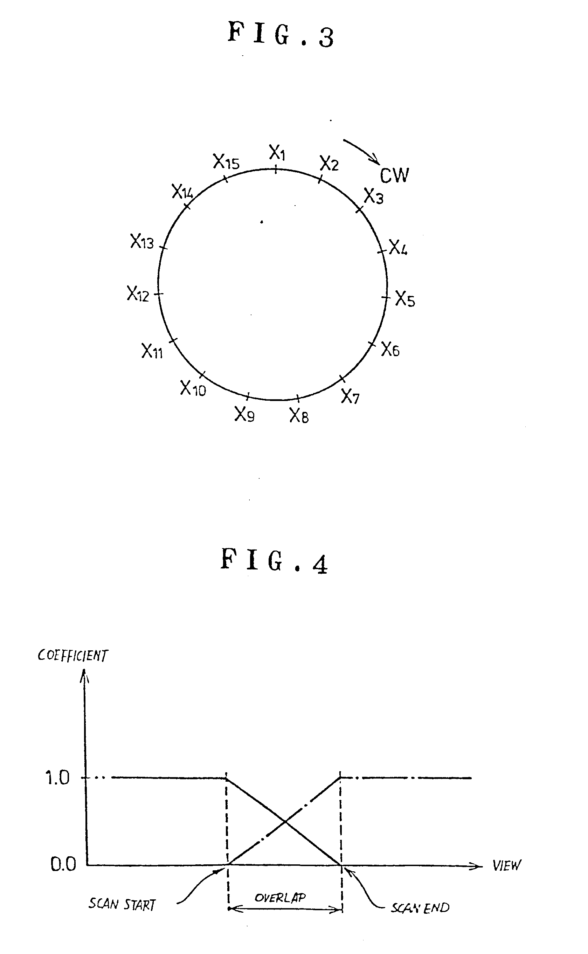

According to these sampling instruction signals, the data collection unit 6 collects scan data at respective rotary positions of the gantry. If a gantry rotary position is expressed by the rotary position of the X-ray tube 1 when the X-ray tube 1 sequentially reaches positions X₁, X₃, ..., X₁₅, X₂, X₄, ..., X₁₄ on the rotary orbit as shown in Figure 3, scan data is collected individually.

These positions on the rotary orbit where data is collected are called sampling points. Accordingly, data collection based on sampling-instruction signals is done by tracing the fifteen sampling points (X₁, X₂, ..., X₁₅) by skipping one point alternately until the data from all sampling points are collected after two rotations.

The data collected by two gantry rotations is done through view-interlacing, which can be achieved by simply rotating the gantry two times in succession. In this way, there are no scan intervals between the first and second rotation like the application modes based on prior art as previously described. Moreover, gantry rotation and the scan data sampling mode need not be changed. In other words, scan data can be collected through view-interlacing under simple control. The image reconfiguration based on such scan data provides images of high resolution with minimized artifacts.

If scan data is collected by rotating the gantry multiple times, the scan data collected during the preceding rotation and following rotation will be mutually set according to the view-interlacing relationship.

Therefore, each combination of scan data obtained through these rotations in the preceding/following relationships will be scan data collected through view-interlacing.

Therefore, if scan data is collected by rotating the gantry multiple times without changing the slice surface of a subject, view-interlaced scan data for the cine-mode can be obtained. If no view-interlaced scan data is needed for the cine-mode, the scan data respectively obtained from each rotation can be used as the cine-mode data. In this case, a cine-mode image with high time resolution can be obtained. In this case, however, the sampling points for the first rotation will be X₁, X₃, ..., X₁₅, or X₁, X₃, ..., X₁₅, X₂ and the interval between sampling points X₁ and X₁₅ or X₁ and X₂ will be one half that of the other intervals. If such data is used for image reconfiguration as it is, artifacts will be created due to an irregular distribution of sampling intervals or views. To prevent this, an overscan method applied to correct body movement is very effective. According to this method, scan data is collected from scanning that slightly exceeds one full rotation so that the data from an overlapped section will be synthesized by padding it for addition as shown in Figure 4. This process reduces the artifacts created by irregular view distribution.

For continuous X-ray radiation, the gantry continues rotary movement while data collection unit 6 integrates the data from the detector. Consequently, resolution in the direction of rotation is deteriorated. For a single rotation scan, the Nyquist frequency in the transfer function of dislocation becomes 1/2 that of the threshold frequency of the transfer function as shown in Figure 5.

However, if two rotations for a view-interlacing scan are done, an overlapped scan occurs between mutually adjacent sampling points on the rotary orbit of the gantry. This causes the Nyquist frequency to be doubled to match the threshold frequency of the transfer function as shown in Figure 6. Therefore, a deteriorated resolution in the direction of rotation can be recovered up to the vicinity of the threshold frequency.

Moreover, the above mentioned scan data collection method has been described with specific values given to the numbers of rotary encoder marker devices and dividing the ratio of the frequency divider circuit for descriptive convenience. This invention, however, is not restrictive regarding such specified values. Moreover, in the above mentioned application mode in which an X-ray tube and detector rotate as a single body, the detection device group may be arranged by fixing them along the circumference, and the X-ray tube can be structured to rotate continuously.

## Claims

1. An X-rays computerized tomographic device comprising:
- a radiation source (1) that rotates continuously in one direction around a subject (3) M multiple times while continuously radiating an X-ray beam along the sections of the subject,
- a radiation detector (2) installed opposite the radiation source (1) with the subject placed therebetween, said radiation detector (2) consisting of multiple detection devices which detect radiation transmitted through the subject,
- data collection means (6, 11 and 15)
(a) comprising a rotary encoder consisting of N·M - 1 units of marker devices and marker detectors, and a frequency dividing means to divide the output signal from the rotary encoder at a rate of 1 /M,
(b) by which sample output signals are generated by the radiation detector (2) if the position of the radiation source (1) matches one of the sampling points arranged at equal intervals on the rotary orbit of the radiation source (1), and
(c) which, if M and N are positive integers, cause data to be collected at N·M - 1 sampling points during the M rotations of the radiation source (1), since data is collected from a forward sampling point after skipping the M - 1 sampling point adjacent to a point from which a data was collected during a specific rotation, and
a computer (7) to which collected data is fed and reconfigured into tomographic images of the subject according to this data.

2. An X-ray computerized tomographic device as described in claim 1, wherein said frequency dividing means output a sampling timing signal.

3. An X-ray computerized tomographic device as described in claim 1 or 2, wherein said tomographic device has a computer that reconfigures a sectional image of the subject based on the data collected during M rotations of the radiation source (1).

4. An X-ray computerized tomographic device as described in claim 1 or 2, wherein said tomographic device has a computer that reconfigures a sectional image of the subject based on the data collected during one rotation of the radiation source (1).

## Patentansprüche

1. Computergestützter Röntgenstrahlentomograph, gekennzeichnet durch:
- eine Strahlungsquelle (1), die kontinuierlich in einer Richtung M mal um einen Gegenstand (3) rotiert, während kontinuierlich eine Röntgenstrahlenbündel entlang den Schnitten des Gegenstandes abgestrahlt wird;
- einen Strahlungsdetektor (2), der gegenüber der Strahlungsquelle (1) so angeordnet ist, daß sich der Gegenstand dazwischen befindet, wobei der Strahlungsdetektor (2) aus mehreren Detektoreinrichtungen besteht, die die den Gegenstand durchdringende Strahlung erkennen;
- einer Datenerfassungseinrichtung (6, 11 und 15)
(a) mit einem umlaufenden Codierer aus N·M - 1 Einheiten von Markiergeräten und Markierdetektoren und einer Frequenzteilereinrichtung zur Teilung des Ausgangssignals umlaufenden Codierer in einem Verhältnis von 1 : M,
(b) in der Abtastausgangssignale vom Strahlungsdetektor (2) generiert werden, wenn die Position der Strahlungsquelle (1) einem der abstandsgleich auf der Umlaufbahn der Strahlungsquelle (1) angeordneten Abtastpunkten entspricht, und
(c) die, wenn N und M positive ganze Zahlen sind, bewirkt, daß Daten in den N·M - 1 Abtastpunkten während der M Umläufe der Strahlungsquelle (1) erfaßt werden, da Daten von einem vorwärts gelegenen Abtastpunkt nach Überspringen des Abtastpunktes M - 1 neben einem Punkt, in dem ein Datum während einer bestimmten Umdrehung erfaßt werden, und durch
einen Computer (7), in den die erfaßten Daten eingespeist und gemäß dieser Daten zu tomographischen Bildern des Gegenstandes wiederaufgebaut werden.

2. Computergestützter Röntgenstrahlentomograph gemäß Anspruch 1, dadurch gekennzeichnet, daß die Frequenzteilereinrichtung ein Abtastabfolgesignal absetzt.

3. Computergestützter Röntgenstrahlentomograph gemäß Anspruch 1 oder 2, dadurch gekennzeichnet, daß der Tomograph über einen Computer verfügt, der auf Basis der während M Umläufen der Strahlungsquelle (1) erfaßten Daten ein Schnittbild des Gegenstandes wiederaufbaut.

4. Computergestützter Röntgenstrahlentomograph gemäß Anspruch 1 oder 2, dadurch gekennzeichnet, daß der Tomograph über einen Computer verfügt, der auf Basis der während eines Umlaufs der Strahlungsquelle (1) erfaßten Daten ein Schnittbild des Gegenstandes wiederaufbaut.

## Revendications

1. Un dispositif tomographique à rayons X assisté par ordinateur comportant :
- une source de radiations (1) qui tourne en continu dans un sens autour d'un sujet (3) de multiples fois M pendant qu'il émet en continu un faisceau de rayons X le long de sections du sujet,
- un détecteur de radiations (2) disposé à l'opposé de la source de radiations (1), le sujet étant placé entre eux, ledit détecteur de radiations (2) étant constitué de multiples dispositifs de détection qui détectent les radiations transmises à travers le sujet,
- des moyens (6,11 et 15) de réception de données
(a) comportant un codeur rotatif constitué par N.M - 1 unités de dispositifs marqueurs et de détecteurs de marqueurs, et des moyens diviseurs de fréquence pour diviser le signal de sortie provenant du codeur tournant à un taux de 1/M,
(b) par lequel des signaux de sortie échantillons sont engendrés par le détecteur de radiations (2) si la position de la source de radiations (1) correspond à l'un des points d'échantillonnage disposés à intervalles égaux sur l'orbite de rotation de la source de radiations (1), et
(c) qui, si M et N sont des nombres entiers positifs, provoque la réception des données en N.M - 1 points d'échantillonnage pendant les M rotations de la source de radiations (1), pendant que les données sont reçues en provenance d'un point d'échantillonnage avant après le passage du point d'échantillonnage M - 1 adjacent à un point à partir duquel une donnée a été reçue pendant une rotation spécifique, et
un ordinateur (7) dans lequel les données reçues sont délivrées et reconfigurées en images tomographiques du sujet conformément à cette donnée.

2. Un dispositif tomographique à rayons X assisté par ordinateur tel que décrit dans la revendication 1, dans lequel lesdits moyens de division de fréquence délivrent en sortie un signal de rythme d'échantillonnage.

3. Un dispositif tomographique à rayons X assisté par ordinateur tel que décrit dans la revendication 1 ou 2, dans lequel ledit dispositif tomographique comporte un ordinateur qui reconfigure une image sectionnelle du sujet sur la base des données reçues pendant M rotations de la source de radiations (1).

4. Un dispositif tomographique à rayons X assisté par ordinateur tel que décrit dans la revendication 1 ou 2, dans lequel ledit dispositif tomographique comporte un ordinateur qui reconfigure une image sectionnelle du sujet sur la base des données reçues pendant une rotation de la source de radiations (1).
